# EUROPEAN PATENT SPECIFICATION

(11) **EP 1 778 567 B1**
(45) Date of publication and mention of the grant of the patent: **03.09.2008**
(21) Application number: 04775320.7
(22) Date of filing: 19.08.2004
(51) Int. Cl.: B65D 85/16, B65D 85/62, A61F 13/15, B65H 3/32

(54) **ARRANGEMENT FOR REMOVAL OF AN ABSORBENT ARTICLE FROM A STACK OF ABSORBENT ARTICLES**
ANORDNUNG ZUR ENTFERNUNG EINES SAUGFÄHIGEN ARTIKELS VON EINEM STAPEL SAUGFÄHIGER ARTIKEL
SYSTEME PERMETTANT DE SORTIR UN ARTICLE ABSORBANT D'UNE PILE D'ARTICLES ABSORBANTS

(43) Date of publication of application: 02.05.2007
(73) Proprietor: SCA Hygiene Products AB, 405 03 Göteborg (SE)
(72) Inventor: RÖNNBERG, Peter, 431 33 Mölndal (SE); WINQVIST, Pontus, 444 60 Stora Höga1 (SE)
(74) Representative: Olsson, Stefan
(86) International application number: PCT/SE2004/001213
(87) International publication number: WO 2006/025769

(56) References cited:
- EP-A1- 0 391 460
- EP-A1- 0 618 148
- EP-A1- 1 153 838
- US-A- 5 163 558
- US-A- 5 361 905
- US-A- 5 377 837
- US-A- 5 514 067
- US-A1- 2004 129 592

## Description

### TECHNICAL FIELD

The invention relates to an arrangement for the removed of absorbent articles from a pack module.

### BACKGROUND

Within the field of absorbent articles, considerable efforts have been made for many years to permit an increase to be achieved in the quantity of manufactured articles, for example babies' diapers or incontinence pads, per unit of volume in connection with warehousing and transport. For example, twice as many babies' diapers are transported per lorry today compared with the number transported on a lorry of similar size several years ago.

Thinner articles with an unimpaired high absorption capacity are one area in which improvements have been made. Greater thinness has been achieved essentially by the introduction of gelling, so-called superabsorbents in an increasingly high concentration into the absorption bodies of the absorbent articles. The articles have been improved in this way with regard to both storage and transport handling. Thinner absorbent articles have also met with a positive response from their users, which has naturally also influenced various manufacturers to channel developments in this direction.

Thinness has also been achieved by nowadays compressing the articles to a significantly greater degree than previously. Patent EP 0,122,042 describes, for example, how to compress absorbent articles in an effective fashion in order to obtain greater thinness with unimpaired or even increased absorption capacity. This patent stipulates that the compression of absorption bodies must take place at a low moisture content in order to retain softness and pliability despite compressing to high density levels (low bulk levels).

More effective means of packing absorbent articles have also been developed. This has involved the increasingly firm compression of the absorbent articles in conjunction with their being placed in a bag, for example.

Described in Patent Application GB 2,264,278 A is a method for the effective compression of a stack, that is to say an individual pack module, of absorbent articles in conjunction with enclosing the articles in a sleeve. The volume of the pack is reduced by compressing a stack of folded absorbent articles together with a two-part packing wrapper of the sleeve type. The absorbent articles are finally secured in their compressed state by attaching the two parts of the packing wrapper to one another while the absorbent articles are still subject to external compression.

Patent EP 0,780,325 describes a pack in which the absorbent articles are folded and oriented head-to-foot inside the pack. The head-to-foot orientation of folded articles means that some of the articles in each packing stack exhibit their folded parts at the surface of the stack that faces towards the top side of the pack, and the remaining articles exhibit their folded parts at the downward-facing surface of the stack. This way of arranging a packing stack means that the distribution of material between the top surface and the bottom surface of the packing stack is more uniform, so that the stack can be compressed more firmly, and so that the volume of the pack can be reduced.

In the Swedish Patent Application with application number SE-0303557-3, the folded adsorbent articles in a packing stack are displaced in relation to one another. By causing the articles to be displaced in relation to one another, the folded areas of the articles are protected in conjunction with compression, so that the articles can be compressed more firmly in connection with their introduction into a bag, for example.

Every alternate article is preferably displaced in a direction essentially perpendicular to the extension length of the fold area, so that the fold area of every alternate article ends up on a first level in the stack of articles, and the fold area of every other alternate article ends up on a second level in the stack. This way of arranging the absorbent articles in the packing stack permits a reduction in the volume of the pack.

Described in the Swedish Patent Application with application number SE-0303560-7 are pack modules in which constituent folded absorbent articles are both arranged in a head-to-foot fashion and displaced in relation to one another, so that the articles can be compressed even more firmly in connection with their packing, for example in a bag.

A feature common to all packs that comprise firmly compressed absorbent articles is the difficulty in removing the first articles from the pack module. The article, in particular the first article, to be removed from the pack is in contact with (an) adjacent article/articles under high pressure and has surfaces which often exhibit relatively high friction with adjacent articles, as a consequence of which the force required to remove the first articles is high. Moreover, the fact that it is difficult to grip the firmly compressed articles when they are to be withdrawn from the pack also makes the removal of articles from the pack more difficult.

Patent US 4,934,535 describes how absorbent articles can be packed firmly compressed in a pack. The patent also describes how a pack containing compressed absorbent articles is executed so that it is both easy to open and permits the easy removal of the first articles from the pack. The ease with which the first articles can be removed from the pack depends on the removal of a large part of the end surface of the pack when the pack is opened. The part of the end surface that is removed in this connection is equivalent to approximately half the height of the stack of absorbent articles situated behind the end surface. The removed part of the end surface is situated in front of the top part of the stack of articles, so that the articles are afforded the possibility of expanding outwards through the opening. In connection with the expansion, the articles are held securely in the lower part of the stack, where the pack still exhibits an intact end surface. The articles in this case expand like a fan in the direction in which the opening is arranged, so that individual articles can be gripped easily and withdrawn from the pack. Articles that are not immediately removed from the pack remain inside the pack because they are held securely in place by the part of the end wall that was not removed at the time of opening the pack, that is to say at the bottom part of the pack.

One disadvantage associated with the design of the pack described in US 4,934,535 is that the stack of absorbent articles swells beyond the initial boundary surfaces of the pack, so that the pack becomes more difficult to handle in its opened state. Another problem which arises is that remaining articles that are only held securely in place in its lower part can easily fall from the pack during handling. This occurs above all after a number of articles has been removed from the pack, that is to say when compressive forces are no longer holding the lower part of the stack in place. A third negative effect of opening the pack by removing a large part of the end surface of the pack is that contaminants can find their way easily into the pack and contaminate unused articles in the pack. A fourth disadvantage is that the solution only functions for the uppermost stack of articles in packs comprising two or more stacks of articles arranged on top of one another. A pack of this kind, comprising several stacks on top of one another, would require some form of opening point in conjunction with each stack of absorbent articles.

Patent Application WO 95/21107 describes a pack for compressed absorbent articles. The pack comprises an openable continuous perforation around the entire pack, so that the perforation extends over the four lateral surfaces of the pack at a distance from the top surface of the pack. The pack is opened by removing the whole of the top part, which removed top part comprises the top surface of the pack and parts of the four lateral surfaces of the pack. After the pack has been opened, the top part of the firmly compressed stack of absorbent articles extends beyond the top edge of the pack. The result is that the top part of the articles included in the stack is permitted to expand like a fan, while the lower part of the stack is retained in place by remaining parts of the pack. Regarding the ease of removing individual articles from the pack, the pack in WO 95/21107 functions in principle in the same way as the pack described in US Patent 4,934,535.

The same advantages and disadvantages that are associated with the pack described in US 4,934,535 also exist in principle for the pack in Patent Application WO 95/21107.

US Patent 5,377,837 describes a bag for firmly compressed absorbent articles, which bag comprises an expansion part that is activated when the bag is opened. The expansion part has been created by the arrangement of a pleat in one of the end surfaces of the bag. The pleat in this case is executed so that it is secured in its inward-folded position until the bag is opened. The securing of the pleat is released in conjunction with opening of the bag, and the bag expands to a certain extent in the direction of compression of the articles, so that the articles are no longer compressed as firmly and individual articles can be removed more easily from the bag. The solution described in US 5,377,837 probably functions in an effective fashion, although the solution involves a significantly more complicated and expensive design of the bag. Another disadvantage is that more material is used to manufacture the bag. Finally, the inward-facing expansion pleat of the bag means that the bag must be handled with greater care when it is being filled with absorbent articles so that the fold in the expansion pleats is not altered during filling.

A need accordingly remains for an arrangement for the removal of firmly compressed absorbent articles from a pack, which arrangement does not involve the use of an unnecessary amount of material in the bag.

There also remains a need for an arrangement for the removal of firmly compressed absorbent articles from a pack where articles remaining in the pack are afforded optimal protection.

There is furthermore a need for an arrangement for the removal of firmly compressed absorbent articles from a pack so that the pack and its contents can be handled in a secure fashion after it has been opened and after one or more articles have been removed from the pack.

### DESCRIPTION OF THE INVENTION

An arrangement has been made available by the present invention, however, in which the arrangement comprises at least one separate gripping device intended to influence and, at least partially, to remove at least one absorbent article from a pack module when the gripping device is used.

The gripping loop comprises a strip of material comprising a first surface and a second surface. The first surface of the strip of material is in contact with the absorbent article which the gripping device is intended to influence, so that the influence occurs through friction between the first surface of the strip of material and the absorbent article.

In accordance with one embodiment of the invention, the gripping device extends beyond at least one of the boundary surfaces of the pack module.

In accordance with one embodiment, the pack module comprising the gripping device is arranged in a pack which comprises an opening. The gripping device in this case is arranged on the boundary surface of the pack module that faces towards the opening of the pack.

In accordance with one embodiment, the gripping device comprises a gripping loop.

In accordance with one embodiment, the second surface of the strip of material, that is to say the surface that faces away from the absorbent article which the gripping device is intended to influence, exhibits lower friction with adjacent absorbent articles in the pack module than the friction between the first surface of the strip of material and the absorbent article which the gripping device is intended to influence.

In accordance with one embodiment, the coefficient of friction between the absorbent article which the gripping device is intended to influence and the gripping device is at least 0.1 higher than the coefficient of friction between the gripping device and adjacent absorbent articles.

In accordance with one embodiment, the strip of material consists of a laminate, which laminate comprises a low-friction layer which, when using the gripping device, is intended to be arranged remotely from the absorbent article which the gripping device is intended to influence.

In accordance with one embodiment, the strip of material comprises a first end area and a second end area, which first end area of the strip of material is arranged on the side of the absorbent article which is intended to be influenced by the gripping device, and which second end area of the strip of material is arranged on the opposite side of the article, so that the part of the strip of material situated between the end areas constitutes the gripping loop. The expression 'sides of the article is intended to denote outward-facing sides of the article when it is configured for packing in a pack module, so that the sides face towards adjacent articles in the pack module. If the article which the gripping device is intended to influence consists of one of the articles that are arranged in an outermost position in the pack module, only one of the sides will face towards an adjacent article in the pack module, so that the opposite side of the article will constitute one of the outward-facing boundary surfaces of the pack module.

In accordance with one embodiment, the first end area of the strip of material covers essentially the whole of the side of the absorbent article which the gripping device is intended to influence, and the second end area of the strip of material covers essentially the whole of the opposite side of the article.

In accordance with one embodiment, the gripping device is attached to the absorbent article which the gripping device is intended to influence.

In accordance with one embodiment, at least parts of the gripping device can be removed from the absorbent article to which the gripping device is attached.

In accordance with one embodiment, the gripping device is attached by means of pressure-sensitive adhesive to the absorbent article which the gripping device is intended to influence, so that the entire gripping device is capable of being removed from the absorbent article.

In accordance with one embodiment, the gripping device constitutes an information carrier, in which case the information can consist of text, images and/or symbols, for instance.

In accordance with one embodiment, the absorbent article which the gripping device is intended to influence consists of an absorbent article which differs from other absorbent articles in the pack module in respect of its design and/or in respect of its constituent component parts.

In accordance with one embodiment, the gripping device comprises information about an absorbent article, with the article that the gripping device is intended to influence consisting of an example of the absorbent article to which the information relates. The arrangement in accordance with the embodiment is particularly attractive in connection with the launch of upgraded articles, the next size in a particular range of articles, or the like. Opportunities are available both for providing information relating to a new, upgraded article / next size in the range and for providing the user with a sample of the article.

### BRIEF DESCRIPTION OF THE FIGURES

- Figure 1: illustrates a disposable diaper intended for infants, which can be included in a pack in accordance with the invention.
- Figure 2: illustrates schematically a first example of how a diaper can be folded in a pack module in accordance with the invention.
- Figure 3a: illustrates a pack module comprising an arrangement for removing a diaper from a pack module in accordance with one embodiment of the invention.
- Figure 3b: illustrates a diaper contained in the pack module in Fig. 3a, which diaper comprises the arrangement for removing the diaper from the pack module.
- Figure 3c: illustrates a diaper contained in the pack module in Fig. 3, which diaper comprises an alternative embodiment of an arrangement for removing the diaper from the pack module.
- Figure 3d: illustrates an alternative embodiment of a pack module comprising a gripping device so arranged as to influence two diapers.
- Figure 4: illustrates an unopened pack comprising a pack module comprising an arrangement for removing a diaper from the pack in accordance with the invention.
- Figure 5: illustrates the pack in accordance with Fig. 4 in the opened state.
- Figure 6a: illustrates a pack module comprising an arrangement for removing a diaper from a pack module in accordance with an alternative embodiment of the invention.
- Figure 6b: illustrates a diaper contained in the pack module in Fig. 6a, which diaper comprises an alternative arrangement for removing a diaper from the pack module.
- Figure 6c: illustrates a diaper contained in the pack module in Fig. 6a, which diaper comprises a second alternative arrangement for removing a diaper from the pack module.
- Figure 7: illustrates a diaper comprising an alternative arrangement for removing a diaper from the pack module.
- Figure 8a: illustrates a cross section through a pack comprising an alternative arrangement for removing one or more diapers from a pack.
- Figure 8b: illustrates a cross section through a pack comprising an alternative arrangement for removing one or two diapers from a pack.

### DESCRIPTION OF THE ILLUSTRATIVE EMBODIMENTS

The invention relates to an arrangement for removing absorbent articles from a pack comprising at least one pack module, in conjunction with which the pack module comprises firmly compressed absorbent articles.

Absorbent articles contained in a pack module comprising an arrangement for removing the articles from the pack module may consist of so-called all-in-one diapers, pant diapers or belt diapers. The articles can also consist of baby diapers intended for incontinent children or incontinence pads intended for incontinent adult wearers. The invention can also be applicable to packs comprising absorbent articles intended for menstruation.

So-called pant diapers are characterized above all in that they have already been folded at the time of manufacture about an essentially transverse fold line in the crotch area, after which they have been joined together at the side parts. Diapers of this type are intended to be put on a wearer precisely like a pair of underpants, that is to say they are passed over the wearer's legs.

Belt diapers are **characterized in that** they comprise a, in relation to the absorbent part of the diaper, transverse belt attached to either the front or the rear transverse edge of the diaper. When putting on a belt diaper of this kind, the belt is fitted as a first step around the wearer's waist. The absorbent part of the diaper hangs loosely from the belt at this point. The absorbent part of the diaper is then passed between the wearer's legs and is fastened to the belt, for which purpose the belt comprises fixing surfaces intended to adhere strongly to fixing devices arranged on the absorbent part of the diaper adjacent to its free transverse edge.

Diaper inserts are characterized in that they are intended to be placed inside retaining pants or, for example, a pair of underpants, with the inserts lacking special attachment devices.

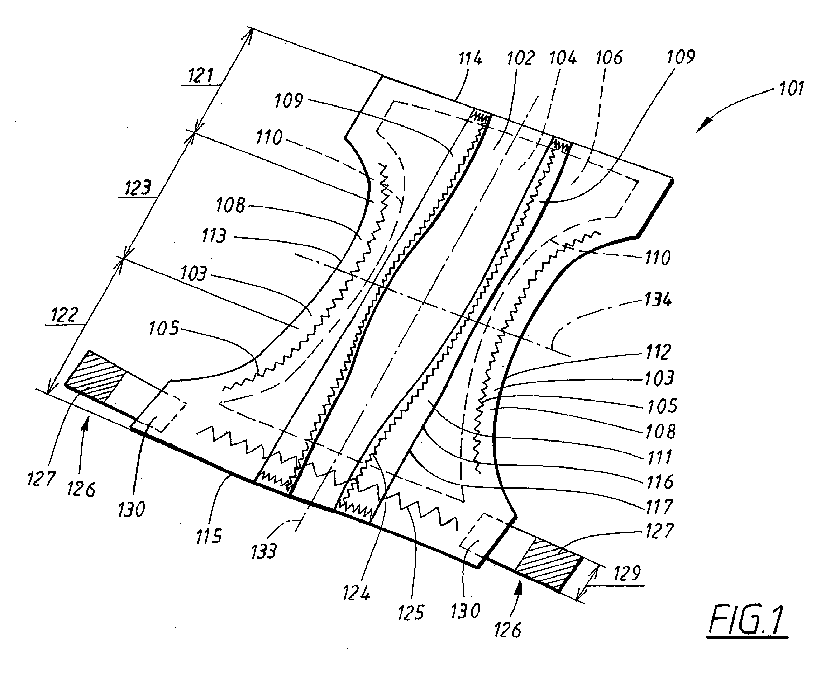

Fig. 1 illustrates the main components of a diaper 101 which can be included in a pack in accordance with the invention.

The diaper 101 is an open baby's diaper of the so-called all-in-one type. The diaper 101 in this case is not joined together in the waist part at the time of sale, but is intended instead to be placed around the infant's abdomen and then joined together around the infant's waist.

The diaper 101 essentially exhibits the form of an hourglass and in this case exhibits longitudinal edges 112, 113, a front transverse edge 114 and a rear transverse edge 115. The diaper 101 also exhibits a front end portion 121, a rear end portion 122 and a narrower crotch portion 123 located between the end portions 121, 122. The crotch portion 123 is intended to be situated in the narrowest area between the wearer's thighs during use.

A longitudinal line of symmetry 133, which divides the article into a right half and a left half, is drawn in Fig. 1. The two halves in this case are laid out symmetrically to either side of the line of symmetry 133. Also drawn in Fig. 1 is a transverse centre line 134. The transverse centre line 134 in this case divides the diaper 101 into a front part and a rear part, whereby the length of the front part and the rear part is essentially the same in the longitudinal direction of the diaper 101.

The diaper 101 comprises a liquid-permeable covering layer 102 arranged over the surface of the diaper 101 which is intended to face towards the wearer when it is being worn, a backing layer 104 arranged over the surface of the diaper which is intended to face away from the wearer when it is being worn, an absorption body 106 enclosed between the liquid-permeable covering layer 102 and the backing layer 104, and side flaps 103 arranged outside the absorption body 106.

The liquid-permeable covering layer 102 of the diaper 101 extends beyond the absorption body 106 along the whole of the periphery of the absorption body 106. The liquid-permeable covering layer 102 can consist of any material that is suitable for the purpose. Examples of commonly encountered liquid-permeable covering materials include non-woven textile materials, known as non-woven materials, perforated plastic films, plastic or textile meshes, and liquid-permeable foam layers. Liquid-permeable covering materials, which consist of continuous thin fibres extending for the most part in the longitudinal or transverse direction of the product, are also found. Laminates consisting of two or more of the above-mentioned possible covering materials are also commonly encountered, as are covers consisting of different materials in different parts of the surface.

Absorbent articles comprising absorption bodies 106, which exhibit particularly high strength and resistance to wear, are even capable of functioning without the need for any additional liquid-permeable covering layer on the side of the article that faces towards the wearer when the article is being worn.

The backing layer 104 also extends beyond the absorption body 106 along the whole of the periphery full extent of the absorption body 106. The backing layers that are normally present on absorbent articles are usually liquid-impermeable, although other types of backing layer are found. The backing layer 104 can consist of a range of different materials. The backing layer 104 most commonly consists of a thin, liquid-impermeable plastic film, although it is also possible to use other types of liquid-impermeable material, such as non-woven material, which have been made liquid-impermeable for example by the use of plastic coating, liquid-impermeable foam layers, liquid-impermeable adhesive or the like. The backing layer 104 can also consist of a vapour-permeable material. Laminates consisting of at least one liquid-impermeable material are also found. These laminates commonly consist of a liquid-impermeable material acting as a liquid barrier and a more textile-like material arranged on the side of the article that faces away from the wearer when the article is being worn, as a consequence of which the outside of the article more closely resembles a piece of cloth.

The liquid-permeable covering layer 102 and the backing layer 104 are attached to one another outside the absorption body 106 along the full periphery of the absorption body 106.

The liquid-permeable covering layer 102 and the backing layer 104 can be attached to one another by a number of different means. Examples of attachment means include gluing, thermo bonding, ultrasonic welding or the like.

Elastic members 105 are arranged outside the absorption body 106 in those parts of the side flaps 103 of the diaper 101 which run essentially in the longitudinal direction of the diaper 101. The elastic members 105 function as leg elastics with the task of preventing liquid and excrement from leaking out past the longitudinal edges 112, 113 of the diaper 101, and in this way form outer liquid barriers 108 together with the surrounding layer. The elastic members 105 consist of one or more elastic threads which, in their stretched state, have been applied between the liquid-permeable covering layer 102 and the backing layer 104, at least in the crotch area 123 of the diaper 101. The elastic members 105 are attached to the backing layer 104 and the covering layer 102 by gluing, ultrasonic welding or the like.

In alternative embodiments, the elastic members can be arranged on the side of the side flaps 103 that is intended to face towards the wearer when the article is being worn or on the opposite side of the side flaps, and in this case they are naturally only attached to the covering layer 102 and the backing layer 104 respectively.

The elastic members in alternative embodiments can consist of elastic tape material, for example foam material.

The hourglass-shaped absorption body 106 can be constructed from one or more layers of cellulose fluff pulp. The cellulose fluff pulp can be mixed in this case with fibres or particles of a high-absorbency polymer material of the kind which, when absorption takes place, chemically bonds large quantities of liquid to form a liquid-containing gel. The absorption body 106 can also contain a high-absorbency polymer material arranged in a layer inside the absorption body or in conjunction with the surface or surfaces of the absorption body. It is also possible to include additional components in the absorption body 106 in order to improve the characteristics of the absorption body 106. Examples of components of this kind include binding fibres, different types of liquid-distributing layers or fibres, form-stabilizing components, reinforcing fibres or the like. The absorption body 106 can naturally also consist of other types of absorption material, such as absorbent non-woven materials, absorbent foams, textile materials, peat or mixtures of different kinds of absorption material.

Special layers with the ability rapidly to receive relatively large quantities of liquid and to retain this liquid temporarily and subsequently to release the temporarily stored liquid to other parts of the absorption body 106 can also be included in diapers of the prescribed kind. Receiving layers of this kind are normally arranged for this purpose between the liquid-permeable covering layer 102 of the diaper 101 and the absorption body 106. No receiving layer is drawn in Fig. 1.

As an additional means of preventing liquid or faeces from leaking out past the lateral edges 112, 113 of the diaper 101, the diaper 101 is provided with internal side leakage barriers 109 on the side intended to face towards the wearer when the article is being worn. The internal side leakage barriers 109 are arranged adjacent to the longitudinal edges 110 of the absorption body 106 and extend essentially in the longitudinal direction of the diaper 101. Each internal side leakage barrier 109 is executed as a separate strip 111 of material, which exhibits two essentially parallel longitudinal edges 116, 117. The strip 111 of material is double-folded, wherein the longitudinal edges 116, 117 of the strip 111 of material are arranged next to one another. The edges 116, 117 of the strip 111 of material are fastened to the covering layer 102 and form the fixed edge of the side leakage barrier. The folded edge of the strip 111 of material forms the free edge of the side leakage barrier 109.

The internal side leakage barriers 109 are folded down and fastened to the covering layer 102 on the front end portion 121 and the rear end portion 122 of the diaper 101.

The internal side leakage barriers 109 contain elastic elements 124 attached to the internal side leakage barriers 109 in a pre-stressed state. The elastic elements 124 are preferably arranged adjacent to the free edges of the internal side leakage barriers 109. When the pre-stressed elastic elements 124 are released, they contract together with the free edges of the side leakage barriers 109, thereby causing the internal side leakage barriers 109 to be brought into a raised configuration remote from the liquid-permeable covering layer 102, at least, in the crotch portion 123 of the diaper 101, where the side leakage barriers 109 are not folded down and fastened to the covering layer 102.

The rear and/or front parts of the diaper 101 can also be provided with a so-called waist elastic 125, which consists of elastic members arranged along the front transverse edge 114 and/or the rear transverse edge 115 of the diaper 101 in order to give the diaper 101 a soft and pliable closure around the wearer's waist. In the described illustrative embodiment, only the rear end portion 122 of the diaper 101 is provided with a waist elastic 125 in the form of a thin strip of an elastic foam material that is attached with adhesive between the backing layer 104 and the liquid-permeable surface layer 102. The waist elastic 125 is applied in the stretched state between the layers 102, 104 in order to achieve a holding force which stretches the diaper 101 around the wearer's waist.

Two soft and inelastic attachment flaps 126 are arranged on the rear end portion 122 for the purpose of securing the diaper 101 around a wearer. One attachment flap 126 is arranged for this purpose on each lateral part of the rear end portion 122. When the article is being worn, the attachment flaps 126 fasten the rear end portion 122 to the front end portion 121 through the attachment flaps 126 exhibiting fixing devices 127, which can be attached to a receiving part arranged on the front end portion 121 of the diaper 101. The attachment flaps 126 are conveniently executed from a very soft and inelastic material, for example from a single non-woven layer or a laminate.

The attachment flaps may be elastic in alternative embodiments.

The fixing devices 127 preferably consist of male parts made of a Velcro^{®} material and are attached to the attachment flaps 126, for example with adhesive, on the side of the attachment flaps 126 which face towards the receiving part when the diaper 101 is being worn.

The receiving part, which is not illustrated in Fig. 1, for the attachment flap 126 consists of a strip of a receiving material adapted for the fixing device 127 of the attachment flap 126. The receiving part extends essentially parallel with the front transverse edge 144 on the side of the diaper which faces away from the wearer when the diaper is being worn, that is to say on the side of the backing layer 104 which faces away from the absorption body 106. The material in the receiving part consists, in the described illustrative embodiment, of a female part made of a Velcro^{®} material and is appropriately executed so that its extent in the longitudinal direction of the diaper 101 corresponds to the width 129 of the attachment flaps 126. The receiving part extends essentially over the full width of the diaper 101 in the transverse direction of the diaper 101.

In alternative illustrative embodiments of a diaper, it is possible to consider the arrangement of separate receiving parts for the respective fixing devices 127, which receiving parts are arranged in conjunction with the longitudinal edges 112, 113 of the diaper on the front transverse edge 114 of the diaper 101.

When putting the diaper 101 on an infant, the diaper 101 is placed between the infant's legs in the infant's crotch. The diaper 101 is then closed around the infant's waist by causing the attachment flaps 126 to overlap the front end portion 121 so that the fixing devices 127 of the attachment flaps 126 can be applied to the receiving part in order to hold the diaper in place.

The attachment flaps 126 are attached to the rear end portion 122 in the attachment areas 130 that are located in the areas of the rear end portion 122 which lie at the lateral edges 112, 133 running in the longitudinal direction. The attachment areas 130 consist of parts of the attachment flaps 126 and those parts of the rear end portion 122 that are attached to one another.

In alternative embodiments, the fixing devices 127 of the attachment flaps 126 can consist of pressure-sensitive adhesive, in which case the receiving part (not shown in Fig. 1) consists of a material to which the selected pressure-sensitive adhesive of the fixing devices 127 can be attached so as to achieve the appropriate joint strength. Combinations of materials are usually selected so that the attachment between the fixing devices 127 and the receiving part can be opened and reclosed to allow the diaper 101 to be checked while it is being worn.

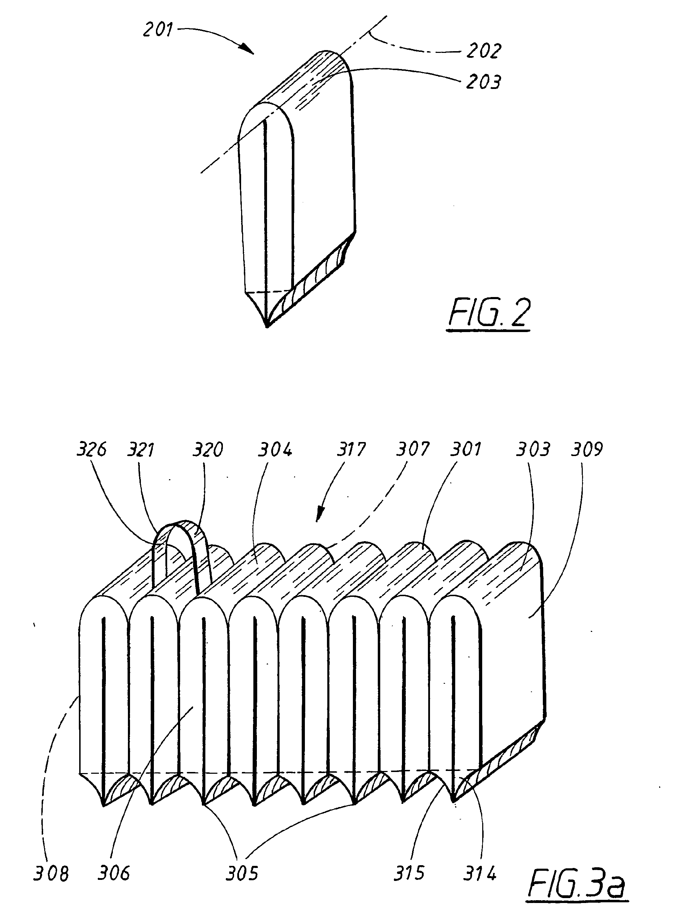

Illustrated in Fig. 2 is a schematic representation of a diaper 201 that is folded in a way which is commonly encountered when diapers 201 are packed. The schematically drawn diaper 201 in Fig. 2 lacks certain components that are present in a diaper in accordance with the invention and are illustrated, for example, in Fig. 1. Examples of components that are not illustrated in Fig. 2 include attachment flaps 126 and waist elastic 125.

The diaper 201 is folded along an essentially transverse fold line 202, essentially in the middle of the diaper 201 in its longitudinal direction. The area adjacent to the fold line 202 constitutes the fold area 203 of the diaper 201. The method of folding diapers 201 along only one essentially transverse fold line 202 is encountered particularly commonly in smaller diapers 201, such as babies' diapers.

Alternative folding procedures are also encountered. It can be mentioned by way of example that larger absorbent articles, such as incontinence pads for adult incontinent users or larger babies' diapers, are often folded along two essentially transverse fold lines. One of the essentially transverse fold lines is arranged in this case in the front half of the incontinence pad, and the other fold line in the rear half of the incontinence pad, which transverse fold lines divide the incontinence pad into three parts of essentially identical length in the longitudinal direction of the incontinence pad. An incontinence pad or diaper folded along two essentially transverse fold lines thus exhibits two fold areas, one adjacent to each fold line. An incontinence pad or a diaper that has been folded along two fold lines exhibits a smaller surface area in its folded state, so that a pack containing the folded incontinence pads can be made easier to handle.

As an alternative, it is also possible to consider folding the incontinence pad or diaper around an additional one or more fold lines.

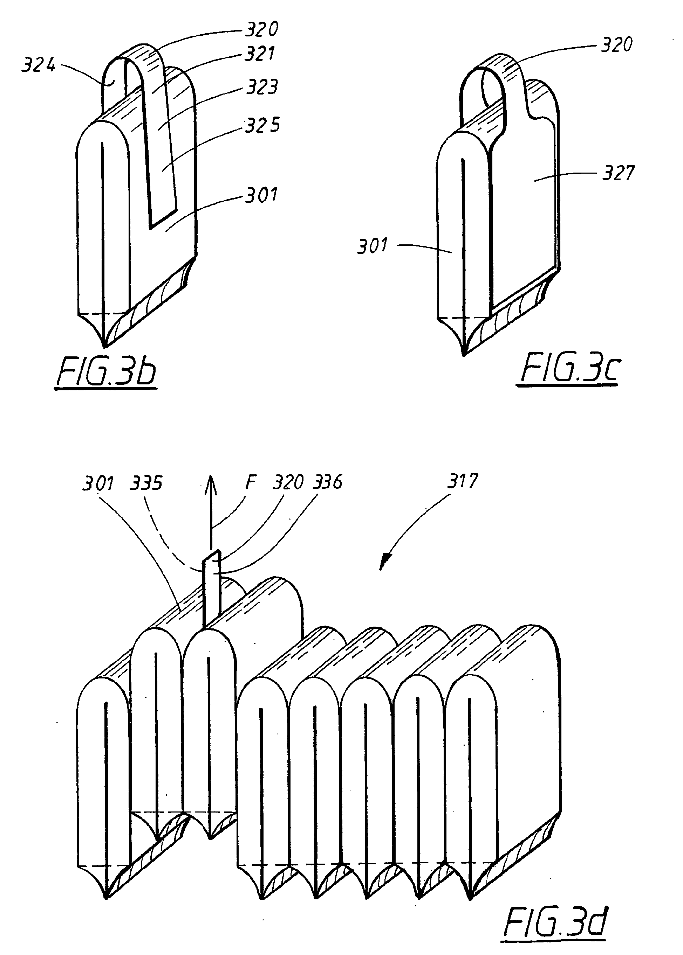

Figure 3 illustrates how diapers 301, folded in accordance with Fig. 2, are configured in a pack module 317 in accordance with a first embodiment of the invention. The pack module 317 comprises eight diapers 301, although it can naturally contain more or fewer diapers 301. The pack module 317 exhibits essentially rectangular parallelepipedic form comprising six outward-facing boundary surfaces. All the diapers 301 in the pack module 317 face the same way in the pack module 317, so that the fold areas 303 of all the diapers 301 are arranged at the same boundary surface 304 in the pack module 317. The front and rear transverse edges 314, 315 of the diapers 301 are arranged at the opposite boundary surface 305 of the pack module 317. The pack module 317 also includes a front boundary surface 306 and a rear boundary surface 307, which boundary surfaces 306, 307 are essentially perpendicular to the boundary surfaces 304, 305. The pack module 317 also includes a first end surface 308 and a second end surface 309, in which case these also constitute boundary surfaces for the pack module 317.

The pack module 317 is characterized primarily in that it comprises at least one gripping device 320. The gripping device 320 can be executed in many different ways and is intended primarily to facilitate the removal of a diaper from a pack module 317, for which purpose it consists appropriately of some form of gripping flap or gripping loop which projects beyond one of the boundary surfaces of the pack module 317. It is possible to remove at least one diaper 301, at least partially, from the pack module 317 by actuating the gripping device 320 by pulling it. The use of a gripping device 320 is particularly advantageous when the first diaper 301 is to be removed from a firmly compressed pack module 317. After removing the first diaper 301, the compression of the diapers 301 in the pack module 317 is reduced, so that the need for additional gripping devices is reduced. It is possible, however, to consider pack modules 317 containing several gripping devices 320, especially if the pack module 317 contains a large number of diapers 301 and is extremely firmly compressed. The gripping device 320 illustrated in Fig. 3a is intended to influence diaper 301 number two from the left in the pack module 317. The gripping device 320 can naturally be intended to influence any diaper in pack module 317. The gripping device 320 is connected to the diaper 301 that is intended to be removed as the first of all the diapers 301 from the pack module 317, that is to say the diaper 301 to be removed when the pack module 317 is compressed to its maximum extent and when the pressure from adjacent diapers 301 in the pack module 317 is at its greatest.

The function of the gripping device 320, in accordance with the first embodiment, is dependent on friction between the gripping device 320 and the outward-facing surface of the folded diaper 301, and on the fact that surrounding diapers 301 press the gripping device 320 against the diaper 301 to which the gripping device is attached. If the gripping device 320 is attached to one of the outermost diapers 301 in the pack module 317, the function of the gripping device 320 will depend on the pressure from the nearest diaper 301 and the end pressure from the packing wrapper in which the pack module 317 is packed.

In alternative embodiments, the function of the gripping device 320 can be dependent on the fact that the gripping device is attached to the outward-facing surface of the folded diaper 301, as described below. The attachment can be separable, in which case the entire gripping device 320 can be removed from the diaper 301 before the diaper 301 is put on a wearer. The attachment can also be permanent, in which case it cannot be removed from the diaper 301. The permanently attached gripping device 320 can incorporate tear instructions or the like, so that parts of the gripping device which threaten to interfere with continued use of the diaper 301 can be torn away.

The gripping device 320 is arranged in such a way in relation to the diaper 301 that a gripping loop 326 is created between the gripping device 320 and the fold area 303 of the diaper 301, in which case at least one finger can be introduced into the gripping loop 326 when the diaper 301 is to be removed from the pack module 317.

The distance by which the diaper 301 can be removed from other diapers 301 in the pack 317 with the help of the gripping device 320 will differ depending on the distance by which the gripping device 320 extends into the pack module 317 from the boundary surface 304 of the pack module 317 towards the opposite boundary surface 305 of the pack module 317.

If the gripping device 320 extends into the pack module 317 for only a short distance, the pressure of the surrounding diapers 301 against the gripping device 320 will be released after only a short distance, in which case the frictional forces between the gripping device 320 and the diaper 301 to be removed from the pack module 317 will also cease. It is usually sufficient, however, for the diaper 301 to have been removed from the pack module 317 with the help of the gripping device 320 to a sufficient extent to permit the individual diaper 301 to be gripped firmly in order to remove it finally from the pack module 317. If, on the other hand, the gripping device 320 extends as far as the boundary surface 305, pressure from surrounding diapers 301 against the gripping device 320 and the diaper 301 that the gripping device 320 is intended to influence will be retained until the diaper has been removed in its entirety from the pack module 317.

The gripping device 320 extends beyond the boundary surface 304 of the pack module 317, so that it is easy to find where the gripping device is located.

It is possible in alternative embodiments for a number of gripping devices 320 to be present, and for each gripping device to be attached to a specific diaper 301 in the pack module 317.

It is also possible to consider the attachment of the gripping device 320 to two diapers 301, in which case both diapers 301 are removed from the pack module 317 when the gripping device 320 is used.

It is also possible to consider the arrangement of the gripping device 320 in such a way as to influence three or more adjacent diapers 301 in the pack module 317, in which case the gripping device 320 is attached to the diapers 301 which the gripping device 320 is intended to influence which are arranged furthest apart. The diapers 301 arranged between the two diapers 301 arranged furthest apart are then influenced indirectly when the gripping device 320 is used.

The specific diaper 301 in the pack module 317 to which the gripping device 320 is attached, that is to say diaper 301 number two from the left in the pack module 317 in Fig. 3, is illustrated in Fig. 3b. The gripping device 320 comprises a long and narrow strip 321 of material, which strip 321 of material consists of a laminate 323. The first surface 324 of the laminate 323, which faces towards the diaper 301 that is to be removed from the pack module 317, consists of a material which exhibits high friction against the outward-facing surface of the folded diaper 301. The second surface 325 of the laminate 323, which faces towards surrounding diapers 301 in the pack module 317, exhibits low friction with the outward-facing surfaces of the surrounding folded diapers 301. Depending on what material is exhibited by the outward-facing surfaces of the diapers 301, the laminate may present different appearances. In the embodiment described here, the folded diapers 301 exhibit outward-facing surfaces which consist of a non-woven material or the like. The first side 324 of the laminate 323, which is in contact with the diaper 301 to be removed by means of the gripping device 320, also exhibits for this purpose a surface which consists of a non-woven material or the like, in conjunction with which high friction is obtained between the diaper 301 and the first surface 324 of the gripping device 320. The second side 325 of the laminate 323, which consists of a low-friction layer intended to slide against surrounding diapers 301 when the gripping device 320 is used, consists of an essentially smooth plastic surface which exhibits low friction with surrounding diapers 301.

It is possible to consider the use of alternative materials in alternative embodiments, in which the function also depends on high friction between the gripping device 320 and the diaper 301 to be removed from the pack module 317 and on lower friction between the gripping device 320 and adjacent diapers 301. For example, the strip 321 of material which comprises the gripping device 320 can consist solely of a plastic film, a strip of paper or the like. Such alternative materials for the gripping device 320 sometimes exhibit the same frictional characteristics on both sides, which can also cause the diapers 301 that are situated closest to the diaper 301 that the gripping device 320 is intended to remove from the pack module 317 to be removed, at least partially, when the gripping device 320 is used. Tests have shown, however, that the problem occurs extremely rarely because the gripping device 320 is in contact with both sides of the diaper 301 that is intended to be influenced by the gripping device 320 while it is in contact with only one side of the immediately adjacent diaper 301. The result is that the force transferred to the diaper that is to be removed by means of the gripping device is twice as great as the force transferred to adjacent diapers 301. It has also emerged that it is easy for a user to push against the adjacent diapers 301 with the fingers of one hand when pulling one diaper 301 from the pack module 317 with the help of the gripping device 320. If the material in the gripping device 320 consists of only a single plastic film, a strip of paper or the like, it is also possible to treat the material in the gripping device 320 so that it possesses different characteristics with regard to friction on the two sides of the material. The material can be embossed, for example, thereby making the material different on either side, or one side of the material can be roughened. It is also possible to consider coating one side of the material with a high-friction substance having a rubber-like structure or the like. Because the gripping device 320 interacts with the diaper 301 to be removed from the pack module 317 through friction and pressure, the gripping device 320 constitutes a separate component that must be removed immediately after it has been used.

In order to increase the capacity of the gripping device 320 to slide against adjacent diapers 301 and to increase its frictional forces against the diaper 301 to be influenced by the gripping device 320, it is also possible, in addition to selecting a laminate as described above, to increase the active surface 327 of the gripping device 320 as shown in Fig. 3c. The active surface 327 of the gripping device 320 in this case essentially covers both surfaces of the diaper 301 facing towards adjacent diapers 301 in the pack module 317.

The friction for different material combinations, which have been found to function as material for the gripping device, was measured by the method described below.

The principle for measuring the friction of a material combination, that is to say between two specific materials, involves drawing a sledge, the sliding surface of which is constituted by one material in the combination, over a fixed horizontally orientated surface consisting of the second material in the combination, and measuring the force required to draw the sledge.

The apparatus for the friction measurement consists of a horizontally oriented friction table, a sledge, a tensile testing machine (for example an instron) and a thin flexible wire between the tensile testing machine and the sledge. The test equipment also comprises a deflector pulley arranged on the friction table, in conjunction with which the wire runs over the deflector pulley so that the vertical movement of the tensile testing machine is transformed into a horizontal movement parallel with the friction table. The deflector pulley is made from a rigid plastic material and runs very easily around its suspension, in conjunction with which the additional force generated by the friction of the deflector pulley is negligible for the purposes of the friction measurement.

The sledge consists of a rectangular steel plate with a surface area of 40 cm². The underside of the sledge, that is to say the side that is intended to slide against the friction table, is covered with an elastic coating with a thickness of ca. 3 mm intended to distribute the compressive force uniformly over the surface of the sledge in conjunction with friction measurement. The weight of the sledge is 200 grams (± 5 grams). The edges of the sledge that are intended to face in the direction of movement of the sledge during testing are rounded. The thin wire is attached to the front edge of the sledge, that is to say the edge of the sledge that is intended to face forwards in the direction of movement when testing takes place. The other end of the wire is attached to the tensile testing machine via the deflector pulley. The friction table consists of a stable steel plate with a length of 50 cm and a width of 15 cm. The deflector pulley is arranged on the front edge of the steel plate, and the deflector pulley is positioned so that the wire runs along the line of movement of the tensile testing machine when the wire runs over the deflector pulley.

Test pieces of one of the materials in the material combination intended to cover the underside of the sledge were stamped out with dimensions of 65 x 100 mm. Test pieces of the second material in the material combination intended to cover the friction table were also stamped out with dimensions of 200 x 150 mm. The materials were conditioned at 50 ± 5% r.h. and 23°C for a period of 4 hours before the start of testing.

In conjunction with all handling of the test materials, it is important to handle the test pieces with the greatest care and as little as possible in order to avoid fingerprints, dust and the like, which can interfere with the test results. It is also important to ensure that every test piece is stamped or cut out so that the friction measurement takes place in the intended direction on the respective test piece. Certain types of material, such as many non-woven materials, exhibit different frictional characteristics in the machine direction and in the transverse direction. The test piece that is intended to be applied to the sledge must accordingly be cut so that the long side (100 mm) of the test piece is parallel with the direction for which it is wished to determine the coefficient of friction, and the test piece intended for the friction table must also be cut with its long side (200 mm) parallel with the direction for which it is wished to determine the coefficient of friction. The test piece intended to be applied to the sledge was folded over the rounded front edge of the sledge, after which the test piece was attached to the top side of the sledge next to the said front edge with tape. The test piece was arranged with its long sides (100 mm) in the direction of movement of the sledge, in conjunction with which a small part of the test piece come to be located behind the sledge. It is important to ensure that the test piece covers the underside of the sledge in its entirety and that it is applied smoothly to the sledge. The test piece intended for the friction table was applied essentially centrally on the friction table, and the long sides (200 mm) of the test piece were arranged parallel with the direction of movement of the sledge. The test piece was fixed by means of tape along the short side of the test piece that is arranged on the rear edge of the friction table, that is to say at the furthest distance from the deflector pulley on the friction table. It is important to ensure that this test piece is also smoothly applied.

The wire was connected to the tensile testing machine via the deflector pulley and was tensioned to 0.05 N, after which the force of the tensile testing machine was set to zero. The tensile testing machine was then started, and the sledge was drawn over the test table. The drawing speed was 100 mm/min, and the total drawing distance was 100 mm. Six samples of each material combination were tested. The mean force (F) required in order to draw the sledge over the friction table at a constant speed was calculated. The starting force, that is to say the increased force required in order to set the sledge in motion, was discounted when calculating the mean force. The sledge was weighed, and its weight (W) was determined to an accuracy of 0.03 g, with the weighing being performed before the test piece was applied to the sledge. The dynamic coefficient of friction µ_{D} of the material combination was calculated according to the formula µ_{D} = F / (W x 9.81). The test method is a modified variant of the test method DIN 53 375.

The following material combinations were tested:
1 Mira air 37 g/m² non-woven plastic laminate from Nuova Pansac / Mira air 37 g/m² non-woven plastic laminate from Nuova Pansac. The non-woven surfaces of the laminates were tested against one another in the direction in which the laminates were produced, that is to say in the machine direction.
2 High Impact 24 g/m² embossed polyethylene (PE) film from Nuova Pansac / High Impact 24 g/m² embossed PE film from Nuova Pansac. Both materials were oriented in the directions in which they were produced.
3 Me 1208, 24 g/m² friction plastic (high-friction side) from Trioplanex / Mira air 37 g/m² non-woven plastic laminate from Nuova Pansac, in which case the non-woven side of the laminate was tested. The non-woven surface of the laminate was tested in the direction in which the laminate was produced. The friction plastic was also oriented in the direction of manufacture during the test.
4 Me 1208, 24 g/m² friction plastic (low-friction side) from Trioplanex / Mira air 37 g/m² non-woven from Nuova Pansac. Both the non-woven surface of the laminate and the low-friction side of the friction plastic were tested in the directions in which they were produced.
The Mira air 37 g/m² laminate and the embossed High Impact 24 g/m² PE film can be purchased from Nuova Pansac S.p.a., V. Le Restelli, 5-20124 Milan, Italy.
Me 1208 24 g/m² plastic film can be purchased from Trioplanex International AB, Box 526, 261 24 Landskrona, Sweden.
The following results were obtained:

| Sample No. | Force (F) | Coefficient of friction (µ_{D}) |
|---|---|---|
| 1 | 0.87 N | 0.44 |
| 2 | 1.03 N | 0.53 |
| 3 | 1.44 N | 0.73 |
| 4 | 0.82 N | 0.42 |

All the materials were found, in the course of practical tests on diapers 301 contained in pack modules 317, to function as gripping devices 320 in which the gripping device 320 influences the diaper 301 by means of friction.

Illustrated in Fig. 3d is an alternative embodiment of a pack module 317 comprising a gripping device 320 intended to influence two diapers 301, in which the first surface 335 of the gripping device 320 influences the diaper 301 that is in contact with the said first surface 335, and the second surface 336 of the gripping device 320 influences the diaper 301 that is in contact with the said second surface 336. The gripping device 320 consists of a strip 321 of material which is arranged between two diapers 301 in the pack module 317, in which case the function of the gripping device 320 is dependent on friction between the gripping device 320 and the two diapers 301 between which the gripping device 320 is arranged. The gripping device is executed so that the coefficient of friction between both surfaces of the gripping device 320 and the surfaces of the adjacent diapers 301 is greater than the coefficient of friction between two diapers 301. In order to achieve a satisfactory function, the difference in the coefficient of friction should be at least 0.1.

When the gripping device 320 is subjected to a tensile force F, the immediately adjacent diaper 301 to either side of the gripping device 320 accompanies the latter from the pack module 317. Figure 3d shows the pack module 317 after the gripping device 320 has partially removed two diapers 301 from the pack module 317. The distance for which the diapers 301 accompany the gripping device 320 when it is used will differ depending on the extent to which the gripping device 320 projects between the diapers 301 in the pack module 317. However, the gripping device 320 must project in between the diapers 301 sufficiently to enable the diapers 301 to be withdrawn from the pack module 317 with the help of the gripping device 320 for a distance which is sufficiently long to permit a secure grip to be effected directly around the two diapers 301 after the gripping device 320 has ceased to function. Final removal of the two diapers 301 from the pack module 317 can then be performed by gripping the two diapers 301 and withdrawing them from the pack module 317. The reason why the diapers 301 can be removed only partially from the pack module 317 if the gripping device 320 projects for only a short distance between the diapers 301 is that the normal force against the surface of the gripping device 320 reduces / ceases when the compressive forces from surrounding diapers 301 in the pack module 317 cease, in conjunction with which the frictional forces are also reduced / cease.

It is even possible to consider an embodiment which also consists of a gripping device 320 which, in the same way as the gripping device 320 in Fig. 3b, is inserted between two diapers 301 in a pack module 317, but where the gripping device 320 is attached to the diapers 301 that are in contact with the two surfaces of the gripping device 320. The gripping device 320 can be securely attached to the diapers or detachably attached, in conjunction with which the attachment can consist of an adhesive connection, thermobonding, ultrasonic welded connection or the like.

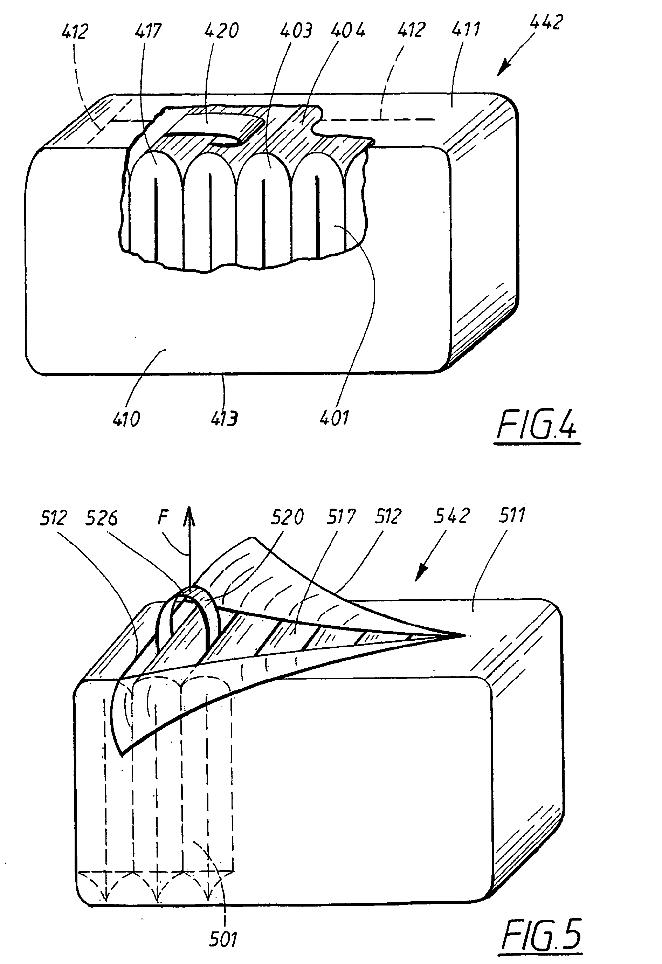

Illustrated in Fig. 4 is a pack 442, into which a pack module 417 comprising a gripping device 420 functioning by means of friction is introduced. The pack module 417 is arranged in the same way as the pack module 317 in Fig. 3. In order to illustrate more clearly how the pack module 417 is arranged inside the pack 442, a part of the front side 410 and the top side 411 of the pack has been removed in Fig. 4. The pack module 417 contains eight diapers 401, but can naturally contain a larger or smaller quantity of diapers 401. The pack 442 consists of a plastic bag, although in alternative embodiments it may consist of a paper bag or some other suitable material. It is also possible to consider a pack 442 consisting of a material combination comprising, for example, a laminate consisting of a plastic film and a paper layer, the plastic film to make the pack 442 tight and the paper layer to give the pack 442 strength.

A pack module 417 can alternatively be enclosed in a sleeve, as described in patent specification WO 93/16925. The pack module 417 provided with a sleeve can then be enclosed in a bag made of a suitable material or supplied without any extra wrapping material. It is also possible to consider vacuum-packing one or more pack modules 417 in accordance with the invention inside an essentially airtight plastic pack.

In alternative embodiments, the pack 442 can contain a number of pack modules 417 arranged side by side or one above the other. It is also possible to consider large packs 442 containing pack modules 417 arranged both side by side and in a number of layers one above the other.

The diapers 401 in the pack module 417 can be arranged in an alternative fashion inside the pack 442; for example, the fold areas 403 of the diapers 401 can face towards the bottom surface 413 of the bag or towards either the front side 410 of the bag or its opposite rear side.

The top side 411 of the pack 442 constitutes the opening side of the pack 442 and comprises a T-shaped perforation 412, which is capable of being broken open and is intended to be broken open when the pack 442 is to be opened.

The gripping device 420, which extends beyond the boundary surface 404 of the pack module 417, lies folded down onto the boundary surface 404 below the opening side of the pack 442.

Illustrated in Fig. 5 is a pack 542 in the opened state. The pack 542 is executed in the same way as the pack 442 in Fig. 4, but the T-shaped perforation 512 of the pack 542 is broken open. In conjunction with the breaking open of the perforation 512, the gripping device 520 has risen up from its folded-down position under the top side 511 of the pack 542. When the first diaper 501 is to be removed from the pack module 517 contained in the pack 542, a finger is expediently introduced through the gripping loop 526 that is formed between the diaper 501 to be removed from the pack 542 and the gripping device 520. The diaper 501 is then removed by the application of a tensile force F to the gripping loop 526.

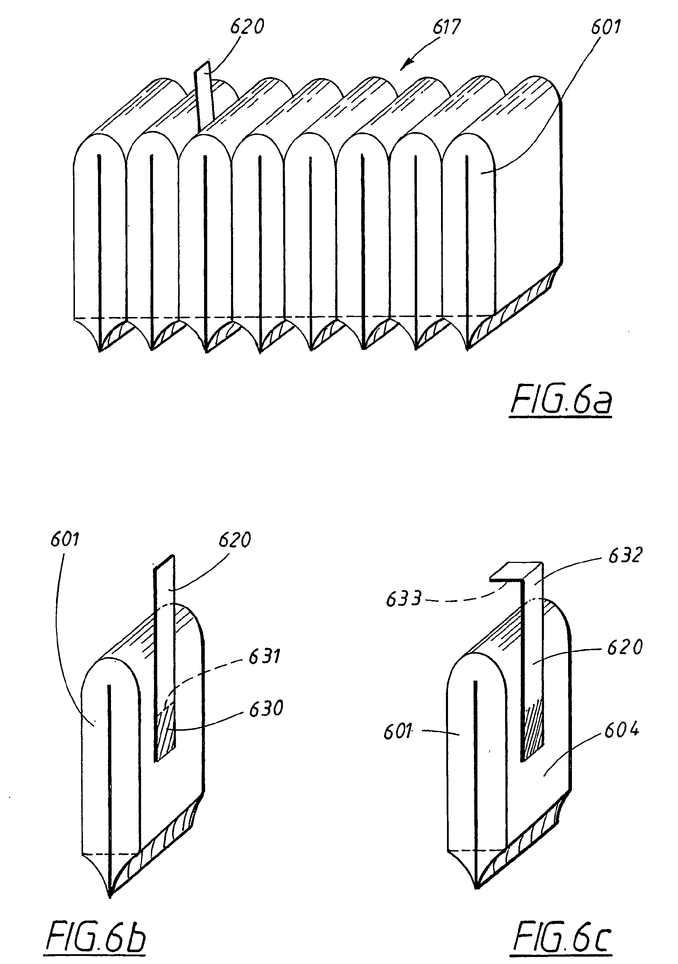

Illustrated in Fig. 6a is an alternative embodiment of an arrangement for the removal of a diaper 601 from a pack module 617, which arrangement comprises a gripping device 620. The gripping device 620 in Fig. 6a is also intended to influence diaper 601 number two from the left in the pack module 617, but it can naturally also be arranged in an equivalent fashion to influence some other diaper 601 in the pack module 617. The gripping device 620 is attached to the side of the diaper 601 that faces towards the centre of the pack module 617, although it can naturally be attached to the opposite side of the diaper 601, or to both sides of the folded diaper facing towards adjacent diapers 601.

Illustrated in Fig. 6b is the specific diaper 601 to which the gripping device 620 is attached. The attachment between the gripping device 620 and the diaper 601 consists of an adhesive joint 630. In alternative embodiments, the gripping device 620 can be attached to the diaper 601 by means of a welded joint or the like, in which case the welded joint can be a thermobonded joint, an ultrasonic welded joint or the like. A perforation 631 is arranged on the gripping device 620, so that the part of the gripping device 620 that is not attached to the diaper 601 can be removed from the diaper 601 when it is to be put on a wearer. The gripping device 620 can also be attached to the diaper 601, in other embodiments, by means of an openable joint of a suitable kind. For example, it is possible to consider executing a welded joint so that it is capable of being opened. An adhesive joint with pressure-sensitive adhesive is another conceivable alternative. An adhesive joint of this kind can be opened after the gripping device 620 has been used, so that the gripping device 620 can be separated from the diaper 601. When a pressure-sensitive adhesive has been selected as the attachment medium between the gripping device 620 and the diaper 601, it is most appropriate for the adhesive to remain on the gripping device 620 after the gripping device 620 has been separated from the diaper 601. It is appropriate for this reason for the receiving surface for the pressure-sensitive adhesive on the diaper 601 to be treated with a release agent in an appropriate fashion, and for the surface of the gripping device 620 not to be treated with a release agent. An adhesive joint with pressure-sensitive adhesive withstands high loads with regard to shearing, and it functions effectively for this reason when the diaper 601 is to be removed from the firmly compressed pack module 617, that is to say when the adhesive joint is exposed to a shearing force. A pressure-sensitive adhesive applied to a surface treated with a release agent exhibits low strength with regard to forces acting perpendicular to the surface of the adhesive, which is an advantage when the gripping device 620 is to be removed from the diaper 601.

The gripping device 620 consists of any suitable material that is capable of being attached to the outward-facing surface of the diaper 601 and exhibits sufficiently high tensile strength in the plane of the material. Suitable materials are plastic films, paper, non-woven materials or the like. It is also possible to consider different laminate structures, in conjunction with which a special layer in the laminate can exhibit good strength characteristics and a second layer in the laminate can exhibit good characteristics with regard to adhesive capacity or weldability to the diaper. One advantage arises if the material in the gripping device 620 exhibits low friction on the side that faces away from the diaper 601, to which the gripping device is attached, since this side of the gripping device 620 is intended to slide against the adjacent diaper 601 in the pack module 617.

An alternative embodiment of the gripping device 620 is illustrated in Fig. 6c. The loose flap 632 of the gripping device 620 is not capable of being detached from the diaper in the same way as the gripping device 620 in Fig. 6b. Instead, the loose flap 632 of the gripping device 620 exhibits an adhesive surface 633 that is capable of attachment to the surface layer of the diaper 601. After the gripping device 620 has been used to remove the diaper 601 from the pack module 617, the free end of the gripping device 620 can be extended out over the surface layer of the diaper and attached to the surface layer of the diaper 601. This embodiment is best suited to diapers 601 that are folded with their waterproof backing layer 604 facing outwards, in which case the gripping device 620 will end up on the outside of the diaper 601 when the diaper 601 is being worn and as such will not interfere with the function of the diaper 601 in relation to comfort or absorption of liquid. The adhesive surface 633 is arranged at the furthermost point on the loose flap 632 of the gripping device 620, in conjunction with which the part that contains the adhesive surface 633 constitutes a double-folded part of the loose flap 632 of the gripping device. The adhesive surface 633 is protected in this way beneath the double-folded part of the loose flap 633 of the gripping device 620. When the adhesive surface 633 is to be used to fix the loose flap 632 of the gripping device 620 to the backing layer 604 of the diaper 601, the double-folded part is opened so that the adhesive surface 633 is exposed and is attached to the backing layer 604 of the diaper 601 as described above. The adhesive in the adhesive surface 633 consists of a pressure-sensitive adhesive, in conjunction with which the part of the gripping device 620 against which the adhesive surface 633 makes contact consists of a surface that is treated with a release agent in an appropriate fashion. The loose flap 632 of the gripping device 620 is in principle executed in the same fashion as a commonly encountered attachment tab for fixing a diaper around the waist of a wearer, in which case the fixing is of the adhesion type. In alternative embodiments, it is also possible to consider that the adhesive surface 633 is covered with a special protective layer treated with a release agent, in conjunction with which the outermost part of the loose flap 632 of the gripping device 620 need not be double-folded. The protective layer in this case must be removed before the loose flap 632 of the gripping device 620 can be fixed to the backing layer 604 of the diaper 601.

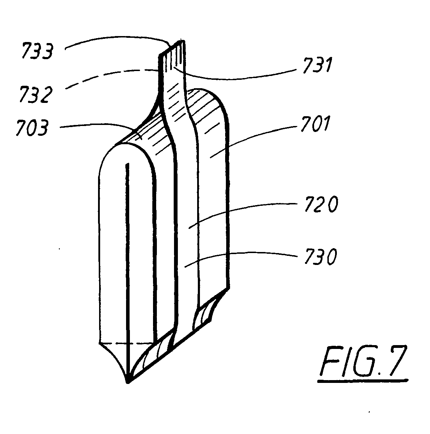

Illustrated in Fig. 7 is an alternative embodiment of an arrangement for removing a diaper 701 from a pack module, which arrangement comprises a gripping device 720. The diaper 701, comprising the gripping device 720, is intended to be present in a firmly compressed pack module in the same way as described above. The gripping device 720 consists of a tape 730, which encloses the diaper 701. Both end areas 731, 732 of the tape 730 extend beyond the fold area 703 of the diaper 701, in conjunction with which the two end areas 731, 732 are appropriately attached to one another. The end areas 731, 732 in this case can be joined together by gluing, thermo bonding, ultrasonic welding or the like. The material in the gripping device 720 consists of a paper strip, although in alternative embodiments it can consist of a plastic strip, a strip of a laminate material or the like. The surface of the gripping device 720 facing away from the enclosed diaper 701 preferably exhibits a surface which exhibits low friction, in which case the diaper 701 and its associated gripping device will slide easily against adjacent diapers at the time of the removal of the diaper 701 from a firmly compressed pack module. The end areas 731, 732 that are joined together and extend beyond the diaper constitute the drawing flap 733 of the gripping device 720, which flap is intended to be used when the diaper 701 is to be removed from a pack module.

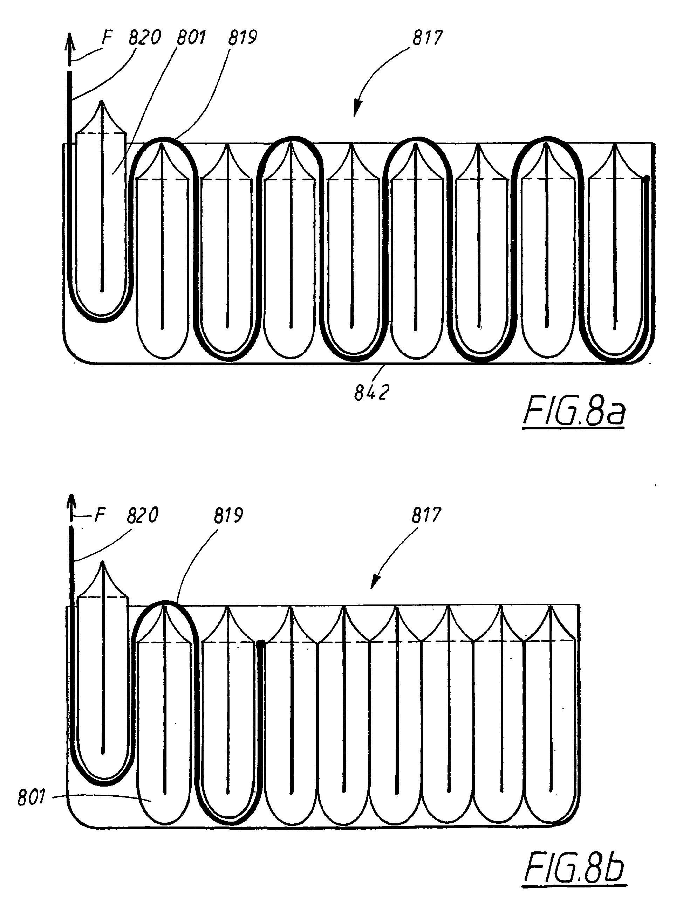

Illustrated in Fig. 8a is an alternative embodiment of an arrangement for removing one or more diapers 801 from a pack module 817, which arrangement comprises a gripping device 820. The pack module 817 is situated inside a pack 842. The Figure shows a cross section through the diapers 801 contained in the pack module 817, and through the pack 842. In order to illustrate more clearly how the arrangement in accordance with the embodiment functions, the normally firmly compressed diapers 801 in the pack module 817 have been drawn apart in the Figure. Finally, the top part of the pack 842, comprising the opening of the pack 842, has been removed in the Figure in order to make the Figure more illustrative.

The gripping device 820 is arranged adjacent to the opening of the pack 842 (not illustrated in Fig. 8a), which gripping device 820 projects beyond the pack module 817 after the pack 842 has been opened. The gripping device 820 constitutes the outermost part of a strip 819 of material that extends through the pack module 817. The strip 819 of material extends in this case in a snaking fashion between the diapers 801 in the pack module 817, in conjunction with which every other diaper 801 is arranged on one side of the strip 819 of material, and every other diaper 801 is arranged on the other side of the strip 819 of material.

The diaper 801 that is situated nearest to the gripping device 820 is arranged on the side of the strip 819 of material that faces towards the opening side of the pack 842, which diaper 801 is removed from the pack module 817 when the gripping device 820 is subjected to a tensile force. When the gripping device 820 is subjected to a tensile force F, a sliding movement takes place between the first diaper 801 and the strip 819 of material, in conjunction with which the first diaper 801 accompanies the device in the direction of movement, when it can be removed from the pack module 817. Frictional forces between the strip 819 of material and the other firmly compressed diapers 801 in the pack module 817 ensure that the strip 819 of material is not pulled out on its own when the gripping device 820 is used, but that the first diaper 801 accompanies the device in the direction of movement. It is advantageous, although not essential, if the strip 819 of material exhibits lower friction on the side that it intended to slide against the diaper 801 that is to be removed from the pack module 817 than on the opposite side of the strip 819 of material. Because the strip 819 of material extends in a snaking fashion between all the diapers 801, it is possible to remove further diapers 801 from the pack module 817. All the diapers 801 that are arranged on the side of the strip 819 of material that faces towards the opening side of the pack 842 can thus be removed one after the other from left to right, that is to say diapers number 1, 3, 5, 7 from the left in the Figure, and so on, when the strip 819 of material is arranged in accordance with Fig. 8a.

In alternative embodiments, it is possible to consider that the strip 819 of material extends only between some of the diapers 801 in the pack module 817, as illustrated in Fig. 8b, in which case only one or a couple of diapers 801 can be removed from the pack module 817 by means of the gripping device 820 and the associated strip 819 of material.

Regardless of whether the gripping device is intended to influence the diaper through frictional forces or through the attachment of the gripping device to the diaper to be influenced, the gripping device is suitable as a carrier of information between the manufacturer and the customer. The gripping device in this case can contain information in the form of text, images, symbols or a combination of at least two of these forms of information. The gripping device can include information about forthcoming new products, for example, or it can serve as discount coupons or the like.

Since the gripping device in the first-mentioned embodiment only influences a specific diaper in the pack module, it is also possible to consider that this specific diaper is a sample diaper, for example a diaper that is about to be launched onto the market. The sample diaper in this case consists of a diaper that differs from other diapers in pack modules with regard to its design or choice of material.

A combination of a gripping device containing information about an upgraded diaper whose market launch is imminent together with a sample of the upgraded diaper that is capable of being influenced by the gripping device is one conceivable embodiment of the invention. Alternatively, it is possible to consider that the diaper that the gripping device is intended to influence consists of the next larger variant of a diaper contained in an entire range of diapers. Consideration can also be given to other situations in which it is wished to provide information about an article, at the same time as it is wished to provide the user with a sample of the article about which information is being provided.

The invention also extends to all conceivable combinations of the described illustrative embodiments.

Furthermore, the invention is not restricted to the above-mentioned illustrative embodiments, but is naturally applicable to other embodiments within the scope of the following Patent Claims.

## Claims

1. Arrangement for removing absorbent articles (101, 201, 301, 401, 501, 601, 701, 801) from a pack module (317, 417, 517, 617, 817), which arrangement comprises at least one separate gripping device (320, 420, 520, 620, 720, 820) intended to influence and, at least partially, to remove at least one absorbent article (101, 201, 301, 401, 501, 601, 701, 801) from the pack module (317, 417, 517, 617, 817) when the gripping device (320, 420, 520, 620, 720, 820) is used, **characterized in that** the gripping device (320, 420, 520, 620) comprises a strip (321) of material comprising a first surface (324) and a second surface (325), with the first surface (324) of the strip (321) of material being in contact with the absorbent article (101, 201, 301, 401, 501, 601) which the gripping device (320, 420, 520, 620) is intended to influence, with the influence occurring through friction between the first surface (324) and the absorbent article (101, 201, 301, 401, 501) or through attachment of the gripping device (620) to the absorbent article (601).

2. Arrangement according to Claim 1, **characterized in that** the gripping device (320, 420, 520, 620, 720, 820) extends beyond at least one of the boundary surfaces (304, 404) of the pack module (317, 417, 517, 617, 817).

3. Arrangement according to Claim 1 or Claim 2, wherein the pack module (317, 417, 517, 617, 817) is arranged in a pack (442, 542, 842) comprising an opening (412), **characterized in that** the gripping device (320, 420, 520, 620, 720, 820) is arranged at the boundary surface of the pack module (317, 417, 517, 617, 817) which faces towards the opening (412) of the pack (442, 542, 842).

4. Arrangement according to any one of the preceding Claims, **characterized in that** the gripping device (320, 420, 520, 620, 720, 820) comprises a gripping loop (326, 526).

5. Arrangement according any one of the preceding Claims, **characterized in that** the influence occurs through friction between the first surface (324) and the absorbent article (101, 201, 301, 401, 501).

6. Arrangement according to Claim 5, **characterized i n that** the second surface (325) of the strip (321) of material which faces away from the absorbent article (101, 201, 301, 401, 501) which the gripping device (320, 420, 520) is intended to influence, exhibits lower friction with adjacent absorbent articles (101, 201, 301, 401, 501) in the pack module (317, 417, 517) than the friction between the first surface (324) of the strip (321) of material and the absorbent article (101, 201, 301, 401, 501) which the gripping device (320, 420, 520) is intended to influence.

7. Arrangement according to Claim 6, **characterized in that** the difference in the coefficient of friction is at least 0.1.

8. Arrangement according to Claim 5 or 6, **characterized in that** the strip (321) of material consists of a laminate (323), with the laminate (323) comprising a low-friction layer arranged remotely from the absorbent article (101, 201, 301, 401, 501) which the gripping device (320, 420, 520) is intended to influence.

9. Arrangement according to any one of Claims 5, 6 or 7, **characterized in that** the strip (321) of material comprises a first end area and a second end area, with the first end area of the strip (321) of material being arranged on the side of the absorbent article (101, 201, 301, 401, 501) which is intended to be influenced by the gripping device (320, 420, 520) and with the second end area of the strip (321) of material being arranged on the opposite side of the article (101, 201, 301, 401, 501), with the sides of the article (101, 201, 301, 401, 501) denoting outward-facing sides of the article (101, 201, 301, 401, 501) when the article (101, 201, 301, 401, 501) is configured for packing in a pack module (317, 417, 517), with the part of the strip (321) of material situated between the end areas constituting the gripping loop (326, 526).

10. Arrangement according to Claim 8, **characterized in that** the first end area of the strip (321) of material covers essentially the whole of the side of the absorbent article (101, 201, 301, 401, 501) which the gripping device (320, 420, 520) is intended to influence, and with the second end area of the strip (321) of material covering essentially the whole of the opposite side of the article (101, 201, 301, 401, 501).

11. Arrangement according to any one of Claims 1-4, **characterized in that** the gripping device (320) is intended to influence two absorbent articles (301), with the first surface (335) of the gripping device (320) being intended to influence one of the said two absorbent articles (301) and with the second surface (336) of the gripping device being intended to influence the other of the said two absorbent articles (301).

12. Arrangement according to any one of Claims 1-4, **characterized in that** the influence occurs through attachment of the gripping device (620) to the absorbent article (601).

13. Arrangement according to Claim 12, **characterized in that** at least parts of the gripping device (620) can be removed from the absorbent article (601) to which the gripping device (620) is attached.

14. Arrangement according to Claim 12 or 13, **characterized in that** the gripping device (620) is attached to the absorbent article (601) which the gripping device (620) is intended to influence by means of pressure-sensitive adhesive, with the entire gripping device (620) being capable of being removed from the absorbent article (620).

15. Arrangement according to any one of the preceding Claims, **characterized in that** the gripping device (320, 420, 520, 620, 720, 820) constitutes an information carrier, with the information being able to consist of text, images and/or symbols, for instance.

16. Arrangement according to any one of the preceding Claims, **characterized in that** the absorbent article (101, 201, 301, 401, 501, 601, 701, 801) which the gripping device (320, 420, 520, 620, 720, 820) is intended to influence consists of an absorbent article (101, 201, 301, 401, 501, 601, 701, 801) which differs from other absorbent articles (101, 201, 301, 401, 501, 601, 701, 801) in the pack module (317, 417, 517, 617, 817) in respect of its design and/or in respect of its constituent component parts.

17. Arrangement according to Claims 15 and 16, **characterized in that** the gripping device (320, 420, 520, 620, 720, 820) comprises information about an absorbent article (101, 201, 301, 401, 501, 601, 701, 801), with the article (101, 201, 301, 401, 501, 601, 701, 801) which the gripping device (320, 420, 520, 620, 720, 820) is intended to influence consisting of an example of the absorbent article (101, 201, 301, 401, 501, 601, 701, 801) to which the information relates.

## Patentansprüche

1. Anordnung zum Entnehmen absorbierender Gegenstände (101, 201, 301, 401, 501, 601, 701, 801) aus einem Gebinde (317, 417, 517, 617, 817), wobei die Anordnung umfasst: wenigstens eine separate Greifeinrichtung (320, 420, 520, 620, 720, 820), die dazu gedacht ist auf wenigstens einen absorbierenden Gegenstand (101, 201, 301, 401, 501, 601, 701, 801) zu wirken und ihn wenigstens teilweise aus dem Gebinde (317, 417, 517, 617, 817) zu entnehmen, wenn die Greifeinrichtung (220, 420, 520, 620, 720, 820) verwendet wird, **dadurch gekennzeichnet, dass** die Greifeinrichtung (320, 420, 520, 620) einen Materialstreifen (321) mit einer ersten Fläche (324) und einer zweiten Fläche (325) umfasst, wobei die erste Fläche (324) des Materialstreifens (321) in Kontakt mit dem absorbierenden Gegenstand (101, 201, 301, 401, 501, 601), auf den die Greifeinrichtung (320, 420, 520, 620) wirken soll, steht, wobei die Einwirkung durch Reibung zwischen der ersten Fläche (324) und dem absorbierenden Gegenstand (101, 201, 301, 401, 501) oder durch Anbringung der Greifeinrichtung (620) an dem absorbierenden Gegenstand (601) erfolgt.

2. Anordnung nach Anspruch 1, **dadurch gekennzeichnet, dass** sich die Greifeinrichtung (320, 420, 520, 620, 720,820) über wenigstens eine der Grenzflächen (304, 404) des Gebindes (317, 417, 517, 617, 817) hinaus erstreckt.

3. Anordnung nach Anspruch 1 oder 2, bei der das Gebinde (317, 417,517, 617, 817) in einer Verpackung (442, 542, 842) mit einer Öffnung (412) angeordnet ist, **dadurch gekennzeichnet, dass** die Greifeinrichtung (320, 420, 520,620, 720, 820) an der Grenzfläche des Gebindes (317, 417, 517, 617, 817) angeordnet ist, die in Richtung der Öffnung (412) der Verpackung (442, 542, 842) weist.

4. Anordnung nach einem der vorstehenden Ansprüche, **dadurch gekennzeichnet, dass** die Greifeinrichtung (320, 420, 520, 620, 720, 820) eine Griffschlaufe (326, 526) umfasst.

5. Anordnung nach einem der vorstehenden Ansprüche, **dadurch gekennzeichnet, dass** die Wirkung durch Reibung zwischen der ersten Fläche (324) und dem absorbierenden Gegenstand (101, 201, 301, 401, 501) erfolgt.

6. Anordnung nach Anspruch 5, **dadurch gekennzeichnet, dass** die zweite Fläche (325) des Materialstreifens (321), die von dem absorbierenden Gegenstand (101, 201, 301, 401, 501), auf die die Greifeinrichtung (320, 420, 520) wirken soll, weg weist, eine niedrigere Reibung gegenüber benachbarten absorbierenden Gegenständen (101, 201, 301, 401, 501) in dem Gebinde (317, 417, 517) aufweist, als zwischen der ersten Fläche (324) des Materialstreifens (321) und des absorbierenden Gegenstands (101, 201, 301, 401, 501) auf den die Greifeinrichtung (320, 420, 520) wirken soll.

7. Anordnung nach Anspruch 6, **dadurch gekennzeichnet, dass** der Unterschied der Reibungskoeffizienten wenigstens 0,1 beträgt.

8. Anordnung nach Anspruch 5 oder 6, **dadurch gekennzeichnet, dass** der Materialstreifen (321) aus einem Verbund (323) besteht, wobei der Verbund (323) eine Lage mit geringerer Reibung umfasst, die vom absorbierenden Gegenstand (101, 201, 301, 401, 501), auf den die Greifeinrichtung (320, 420, 520) wirken soll, entfernt angeordnet ist.

9. Anordnung nach einem der Ansprüche 5, 6 oder 7, **dadurch gekennzeichnet, dass** der Materialstreifen (321) einen ersten Endbereich und einen zweiten Endbereich umfasst, wobei der erste Endbereich des Materialstreifens (321) auf der Seite des absorbierenden Gegenstands (101, 201, 301, 401, 501) auf den die Greifeinrichtung (320, 420, 520) wirken soll, angeordnet ist und wobei der zweite Endbereich des Materialstreifens (321) auf der entgegengesetzten Seite des Gegenstands (101, 201, 301, 401, 501) angeordnet ist, wobei die Seiten des Gegenstands (101, 201, 301, 401, 501) nach außen weisende Seiten des Gegenstands (101, 201, 301, 401, 501) bezeichnen, wenn der Gegenstand (101, 201, 301, 401, 501) zur Verpackung in einem Gebinde (317, 417, 517) ausgebildet ist, wobei der Teil des Materialstreifens (321), der zwischen Endbereichen angeordnet ist, die Griffschlaufe (326, 526) bildet.

10. Anordnung nach Anspruch 8, **dadurch gekennzeichnet, dass** der erste Endbereich des Materialstreifens (321) im Wesentlichen die gesamte Seite des absorbierenden Gegenstand (101, 201, 301, 401, 501), auf die die Greifeinrichtung (320, 420, 520) wirken soll, bedeckt und wobei der zweite Endbereich des Materialstreifens (321) im Wesentlichen die gesamte entgegengesetzte Seite des Gegenstands (101, 201, 301, 401, 501) bedeckt.

11. Anordnung nach einem der Ansprüche 1-4, **dadurch gekennzeichnet, dass** die Greifeinrichtung (320) dazu gedacht ist, auf zwei absorbierende Gegenstände (301) zu wirken, wobei die erste Fläche (335) der Greifeinrichtung (320) auf einen der zwei besagten absorbierenden Gegenständen (301) wirken soll und wobei die zweite Fläche (336) der Greifeinrichtung auf den anderen der zwei besagten absorbierenden Gegenstände (301) wirken soll.

12. Anordnung nach einem der Ansprüche 1-4, **dadurch gekennzeichnet, dass** die Wirkung durch Anbringung der Greifeinrichtung (620) an dem absorbierenden Gegenstand (601) erfolgt.

13. Anordnung nach Anspruch 12, **dadurch gekennzeichnet, dass** wenigstens Teile der Greifeinrichtung (620) von dem absorbierenden Gegenstand (601), an dem die Greifeinrichtung (620) angebracht ist, entfernt werden können.

14. Anordnung nach Anspruch 12 oder 13, **dadurch gekennzeichnet, dass** die Greifeinrichtung (620) durch Haftklebemittel an dem absorbierenden Gegenstand (601) angebracht ist, auf den die Greifeinrichtung (620) wirken soll, wobei die gesamte Greifeinrichtung (620) von dem absorbierenden Gegenstand (620) entfernt werden kann.

15. Anordnung nach einem der vorstehenden Ansprüche, **dadurch gekennzeichnet, dass** die Greifeinrichtung (23, 24, 25, 26, 27, 28) einen Informationsträger bildet, wobei die Informationen aus z. B. Text, Bildern und/oder Symbolen bestehen können.

16. Anordnung nach einem der vorstehenden Ansprüche, **dadurch gekennzeichnet, dass** der absorbierende Gegenstand (101, 201, 301, 401, 501, 601, 701, 801), auf den die Greifeinrichtung (320, 420, 520, 620, 720, 820) wirken soll, aus einem absorbierenden Gegenstand (101, 201, 301, 401, 501, 601, 701, 801) der sich von anderen absorbierenden Gegenständen (101, 201, 301, 401, 501, 601, 701, 801) in dem Gebinde (317, 417, 517, 617, 817) in Bezug auf die Ausgestaltung und/oder in Bezug auf die bildenden Komponententeile unterscheidet, besteht.

17. Anordnung nach Anspruch 15 und 16, **dadurch gekennzeichnet, dass** die Greifeinrichtung (320, 420, 520, 620, 720, 820) Informationen über einen absorbierenden Gegenstand (101, 201, 301, 401, 501, 601, 701, 801) umfasst, wobei der Gegenstand (101, 201, 301, 401, 501, 601, 801), auf den die Greifeinrichtung (320, 420, 520, 620, 720, 820) wirken soll aus einem Beispiel eines absorbierenden Gegenstand (101, 201, 301, 401, 501, 601, 701, 801), auf das sich die Informationen beziehen, besteht.

## Revendications

1. Agencement destiné à retirer des articles absorbants (101, 201, 301, 401, 501, 601, 701, 801) d'un module d'emballage (317, 417, 517, 617, 817), lequel agencement comprend au moins un dispositif de saisie séparé (320, 420, 520, 620, 720, 820) destiné à exercer une action sur au moins un article absorbant (101, 201, 301, 401, 501, 601, 701, 801) et, au moins partiellement, à le retirer du module d'emballage (317, 417, 517, 617, 817) lorsque le dispositif de saisie (320, 420, 520, 620, 720, 820) est utilisé, **caractérisé en ce que** le dispositif de saisie (320, 420, 520, 620) comprend une bande (321) de matériau comportant une première surface (324) et une deuxième surface (325), la première surface (324) de la bande (321) de matériau étant en contact avec l'article absorbant (101, 201, 301, 401, 501, 601) sur lequel le dispositif de saisie (320, 420, 520, 620) est destiné à exercer une action, laquelle action se produisant par frottement entre la première surface (324) et l'article absorbant (101, 201, 301, 401, 501) ou par l'assujettissement du dispositif de saisie (620) à l'article absorbant (601).

2. Agencement selon la revendication 1, **caractérisé en ce que** le dispositif de saisie (320, 420, 520, 620, 720, 820) s'étend au-delà d'au moins une des surfaces limites (304, 404) du module d'emballage (317,417, 517, 617, 817).

3. Agencement selon la revendication 1 ou selon la revendication 2, dans lequel le module d'emballage (317, 417, 517, 617, 817) est disposé dans un emballage (442, 542, 842) comportant une ouverture (412), **caractérisé en ce que** le dispositif de saisie (320, 420, 520, 620, 720, 820) est disposé au niveau de la surface limite du module d'emballage (317, 417, 517, 617, 817) qui est orientée vers l'ouverture (412) de l'emballage (442, 542, 842).

4. Agencement selon l'une quelconque des revendications qui précèdent, **caractérisé en ce que** le dispositif de saisie (320, 420, 520, 620, 720, 820) comporte une boucle de saisie (326, 526).

5. Agencement selon l'une quelconque des revendications qui précèdent, **caractérisé en ce que** l'action se produit par frottement entre la première surface (324) et l'article absorbant (101, 201, 301, 401, 501).

6. Agencement selon la revendication 5, **caractérisé en ce que** la deuxième surface (325) de la bande de matériau (321) qui est orientée à l'écart de l'article absorbant (101, 201, 301, 401, 501) sur lequel le dispositif de saisie (320, 420, 520) est destiné à exercer une action, est doté de moins de frottement avec les articles absorbants voisins (101, 201, 301, 401, 501) présents dans le module d'emballage (317, 417, 517) qu'il n'y a de frottement entre la première surface (324) de la bande (321) de matériau et l'article absorbant (101, 201, 301, 401, 501) sur lequel le dispositif de saisie (320, 420, 520) est destiné à exercer une action.

7. Agencement selon la revendication 6, **caractérisé en ce que** la différence entre les coefficients de frottement est au moins de 0,1.

8. Agencement selon la revendication 5 ou selon la revendication 6, **caractérisé en ce que** la bande (321) de matériau consiste en un stratifié (323), lequel stratifié (323) comprend une couche à faible coefficient de frottement, agencée à l'écart de l'article absorbant (101, 201, 301, 401, 501) sur lequel le dispositif de saisie (320, 420, 520) est destiné à exercer une action.

9. Agencement selon l'une quelconque des revendications 5, 6 ou 7, **caractérisé en ce que** la bande (321) de matériau comporte une première surface d'extrémité et une deuxième surface d'extrémité, la première surface d'extrémité de la bande (321) de matériau étant disposée sur le côté de l'article absorbant (101, 201, 301, 401, 501) qui est destiné à subir l'action du dispositif de saisie (320, 420, 520) et la deuxième surface d'extrémité de la bande (321) de matériau étant disposée sur le côté opposé de l'article (101, 201, 301, 401, 501), les côtés de l'article (101, 201, 301, 401, 501) caractérisant des côtés de l'article (101, 201, 301, 401, 501) qui sont orientés vers l'extérieur lorsque l'article (101, 201, 301, 401, 501) est configuré pour l'emballage dans un module d'emballage (317, 417, 517), la partie de la bande (321) de matériau située entre les surfaces d'extrémité constituant la boucle de saisie (326, 526).

10. Agencement selon la revendication 8, **caractérisé en ce que** la première surface d'extrémité de la bande (321) de matériau recouvre essentiellement la totalité du côté de l'article absorbant (101, 201, 301, 401, 501) sur lequel le dispositif de saisie (324, 420, 520) est destiné à exercer une action, et la deuxième surface d'extrémité de la bande (321) de matériau recouvrant essentiellement la totalité du côté opposé de l'article (101, 201, 301, 401, 501).

11. Agencement selon l'une quelconque des revendications 1 à 4, **caractérisé en ce que** le dispositif de saisie (320) est destiné à exercer une action sur deux articles absorbants (301), la première surface (335) du dispositif de saisie (320) étant destinée à exercer une action sur un desdits deux articles absorbants (301) et la deuxième surface (336) du dispositif de saisie étant destinée à exercer une action sur l'autre desdits deux articles absorbants (301).

12. Agencement selon l'une quelconque des revendications 1 à 4, **caractérisé en ce que** l'action se produit du fait que le dispositif de saisie (620) est assujetti à l'article absorbant (601).

13. Agencement selon la revendication 12, **caractérisé** e n ce qu'au moins des parties du dispositif de saisie (620) peuvent être retirées de l'article absorbant (601) auquel le dispositif de saisie (620) est assujetti.

14. Agencement selon la revendication 12 ou selon la revendication 13, **caractérisé en ce que** le dispositif de saisie (620) est assujetti à l'article absorbant (601) sur lequel le dispositif de saisie (620) est destiné à exercer une action, au moyen d'un adhésif sensible à la pression, l'ensemble du dispositif de saisie (620) pouvant être retiré de l'article absorbant (601).

15. Agencement selon l'une quelconque des revendications qui précèdent, **caractérisé en ce que** le dispositif de saisie (320, 420, 520, 620, 720, 820) constitue un support d'information pouvant consister, par exemple, en un texte, en des images et/ou en des symboles.

16. Agencement selon l'une quelconque des revendications qui précèdent, **caractérisé en ce que** l'article absorbant (101, 201, 301, 401, 501, 601, 701, 801) sur lequel le dispositif de saisie (320, 420, 520, 620, 720, 820) est destiné à exercer une action, consiste en un article absorbant (101, 201, 301, 401, 501, 601, 701, 801) qui diffère des autres articles absorbants (101, 201, 301, 401, 501, 601, 701, 801) présents dans le module d'emballage (317, 417, 517, 617, 817) en ce qui concerne sa présentation et/ou en ce qui concerne ses composants constitutifs.

17. Agencement selon les revendications 15 et 16, **caractérisé en ce que** le dispositif de saisie (320, 420, 520, 620, 720, 820) comporte des informations sur un article absorbant (101, 201, 301, 401, 501, 601, 701, 801), l'article (101, 201, 301, 401, 501, 601, 701, 801) sur lequel le dispositif de saisie (320, 420, 520, 620, 720, 820) est destiné à exercer une action consistant en un exemple de l'article absorbant (101, 201, 301, 401, 501, 601, 701, 801) auquel se rapportent les informations.
